# EUROPEAN PATENT APPLICATION

(11) **EP 2 030 975 A1**
(43) Date of publication of application: **04.03.2009**
(21) Application number: 08159182.8
(22) Date of filing: 27.06.2008
(51) Int. Cl.: C07D 501/60

(54) **CEFDINIR synthesis process**

(30) Priority: 06.08.2007 IT MI20071628
(71) Applicant: ACS DOBFAR S.p.A., 20067 Tribiano (Milano) (IT)
(72) Inventor: Manca, Antonio, 20133, Milano (IT); Filippi, Mauro, 20060, Bettolino Di Mediglia (MI) (IT); Sala, Bruno Maurizio, 20056, Trezzo Sull'Adda (MI) (IT); Monguzzi, Riccardo Ambrogio, 23824, Dorio (LC) (IT); Fogliato, Giovanni, 24030, Barzana (BG) (IT)
(74) Representative: Frignoli, Luigi

(57) **Abstract**

Cefdinir preparation by synthesis of new key intermediates, isolable as variously solvated species complexed with thiophosphoric acid derivatives or with phosphoric acid derivatives.

## Description

This invention describes a new process for synthesizing cefdinir of formula (I) 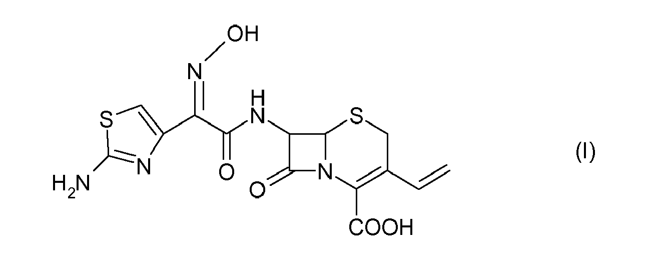 Cefdinir syntheses are described in EP355821 and EP874853.

Specifically, the cefdinir synthesis described in EP874853 comprises isolating an intermediate salified with p-toluenesulphonic acid (p-TsOH) solvated with N,N-dimethylacetamide (DMAC); this salified intermediate, as expressly described, is obtained by condensing 7-amino-3-vinylcephalosporanic acid (7-AVNA) with a particular activated form of (Z)-(2-aminothiazol-4-yl)-2-trityloxyiminoacetic acid, i.e. as mixed anhydride of a thiophosphoric acid derivative, synthesized in accordance with the teachings of EP620228.

The process described in EP874853 also describes the condensation of 7-AVNA trimethylsilyl ester with the 2-benzothiazolyl thioester of (Z)-(aminothiazol-4-yl)-2-trityloxyiminoacetic acid.

In both cases, EP874953 always comprises the addition of p-toluenesulphonic acid to obtain the condensation intermediate of acceptable purity.

The isolation of this salified intermediate necessarily requires diluting the reaction solution, to which p-toluenesulphonic acid has previously been added, with easily flammable toxic organic solvents.

In contrast to that described in the aforesaid known art, the present inventors have surprisingly verified that it is not necessary to use an additional salifying acid, such as p-toluenesulphonic acid, to isolate from an aqueous solution a useful intermediate sufficiently pure for use in cefdinir synthesis.

In this respect, the process claimed herein for obtaining cefdinir of formula (I) describes that a compound of formula (II) 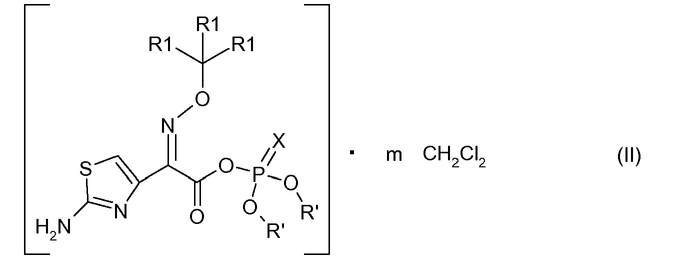 in which the two substituents R1 and R' are independently chosen from the group consisting of phenyl, C1-C4 alkyls, X is chosen from the group consisting of oxygen and sulphur, 0 ≤ m ≤ 1, a compound which in its turn is obtained from the corresponding carboxylic acid of formula (III), 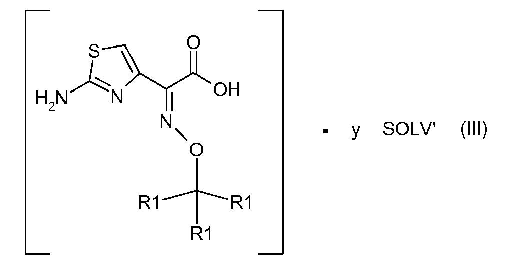 in which SOLV' is a solvent chosen from the group consisting of N,N-dimethylacetamide, N,N-dimethylformamide, N-methylformamide, N-methylacetamide, N-methylpyrrolidone and formamide, R1 has the same meaning expressed in formula (II) and 0 ≤ y ≤ 2, is condensed with a silylester of formula (IV) 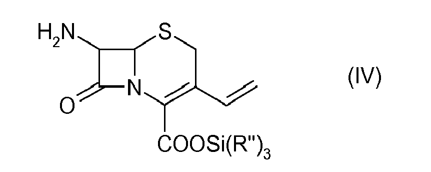 where R" is chosen from the group consisting of equal or different C1-C4 alkyls, to obtain an intermediate, isolated from an aqueous solution which is finally reacted with an acid chosen from the group consisting of borotrifluoride, trifluoroacetic acid, aluminium trichloride, perchloric acid, hydrochloric acid, hydrobromic acid and hydroiodic acid, to generate cefdinir in the corresponding salified form from which cefdinir is obtained.

In addition, when isolated from the reaction environment by filtration, said salified form of cefdinir is then added to a volume of water in which a carbonic acid salt has been previously dissolved, to hence obtain an aqueous solution of cefdinir, of pH between 4.0 and 8.5, which is washed with water-immiscible aprotic organic solvents and decolorized with carbon, to which there has been added a reducing species chosen from the group comprising sulphurated reducing species, themselves chosen from a sulphite, a metabisulphite, a hydrosulphite and a thiosulphite of an alkaline or alkaline-earth metal, organic reducing species and ascorbic acid, or a salt thereof of an alkaline or alkaline-earth metal, to finally precipitate the product at a temperature between 0°C and +44°C, by acidification to a pH between 1.5 and 3.5, then filtering the cefdinir precipitated in this manner and drying it under reduced pressure.

In particular, the process claimed herein comprises the preparation of new key precursors, as intermediates complexed with thiophosphates or phosphates and solvated with N,N-dimethylacetamide, with N,N-dimethylformamide, with N-methylformamide, with N-methylacetamide, with N-methylpyrrolidone and with formamide, described by formula (V) 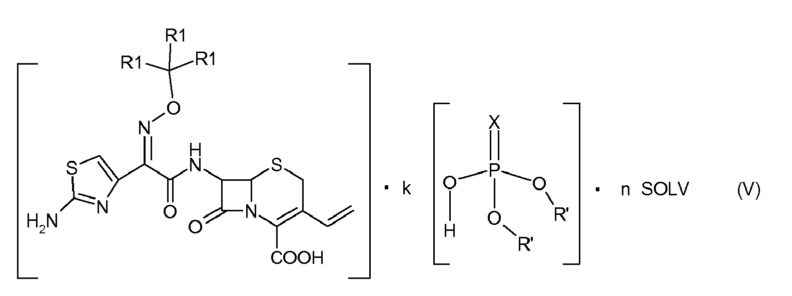 in which the two substituents R' and R1 are independently chosen from the group consisting of phenyl and C1-C4 alkenyls or alkyls, X is chosen from the group consisting of oxygen if the complexing species containing phosphorus is a phosphate, and sulphur if the complexing species containing phosphorus is a thiophosphate, k is a number 0 ≤ k ≤ 1, including fractional, indicating the molar equivalents of the complexing species containing phosphorus, with respect to the cephalosporanic species with which it is associated, while the species SOLV can be a solvent chosen from N,N-dimethylacetamide, N,N-dimethylformamide, formamide, N-methylformamide, N-methylacetamide, and N-methylpyrrolidone, n being hence also understood as a non-whole number 0 ≤ n ≤ 2 indicating the molar equivalents of the solvation species SOLV, with respect to the cephalosporanic species with which it is associated. As already stated, the compound of formula (V) is syhnthesized by initial silylation of 7-AVNA, to obtain the non-isolated compound of formula (IV), a species which is then condensed with a compound of formula (II).

On termination of the condensation between the product (II) and the product (IV), the reaction solution is added to a suitable water volume, the product of formula (V) then being isolated by filtration.

The intermediate (II) is suitably obtained starting from the corresponding carboxylic acid of formula (III), suspended in dichloromethane, possibly in the presence of variable quantities, between 0 and 2 molar equivalents with respect to the substrate (III) used, of an amide chosen from N,N-dimethylacetamide, N,N-dimethylformamide, N-methylformamide, formamide, N-methylacetamide or N-methylpyrrolidone, at a temperature between +10°C and +25°C and, while maintaining the temperature within this range, a catalytic quantity, variable between 1 mol% and 5 mol% with respect to (III), of 1,4-diazabicyclo[2.2.2]octane can be added or another catalyst commonly used for nucleophilic acylations or alkylations chosen, for example, from certain tertiary amines including variously substituted N,N-dialkylaminopyridines, variously substituted N,N-dialkylanilines, alicyclic or heterocyclic tertiary amines, and 1.0-1.5 molar equivalents of a tertiary amine different from that chosen as catalyst, such as triethylamine, pyridine, N-methylmorpholine, diisopropylethylamine or tributylamine. The mixture is agitated until completely dissolved. It is cooled to a temperature less than +10°C and 1.0-1.5 molar equivalents , with respect to (III), of chlorothiophosphonate or chlorophosphonate described in formula (VI) are added, 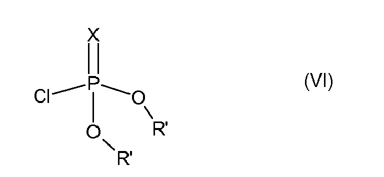 with X equal to sulphur or oxygen, in which the R' groups have the meaning described in formula (V); the mixture is left to react for a time between 1 hour and 5 hours at a temperature between -10°C and +10°C, and then after adding an optional suitable aprotic cosolvent to facilitate precipitation, product (II) is filtered off and dried under reduced pressure to constant weight.

Consequently, the species SOLV described in formula (V) coincides with the solvent used in condensation, hence the resultant species SOLV in product (V) assumes the already described meanings relative to said formula. In the condensation reaction between (II) and (IV), if the amide deriving from the agent used for silylation of the 7-AVNA is different from the amide used as reaction solvent, the species SOLV of the compounds of formula (V) coincides in any event with the amide used as reaction solvent.

Hence the product (V) is obtained in solid form, with high yields, from the organic reaction solution by simple dilution in water, hence avoiding the addition of further organic solvents different from the reaction solvent, as instead is described for isolating the intermediates claimed in EP874853.

From the aforegoing, the present inventors have therefore verified that the process for obtaining cefdinir can be advantageously implemented via synthesis of the intermediates of formula (V), as described in the following scheme 1

Scheme 1 (III) + (VI) ----> (II)

7-AVNA ----> (IV) non-isolated

(IV) non-isolated + (II) ----> (V)

(V) ----> (I)

in which the product (IV),, once formed, is not isolated to proceed directly to the reaction with the separately prepared intermediate (II).

The process comprises initial silylation of 7-AVNA, to obtain the silylester (IV), by suspending the 7-AVNA at ambient temperature in 4-15 volumes, with respect to the weight of the chosen substrate, of a solvent selected from N,N-dimethylacetamide, N,N-dimethylformamide, N-methylacetamide, N-methylformamide, N-methylpyrrolidone and formamide. The mixture is cooled to a temperature (indicated hereinafter by the single letter "T") between 0°C and +25°C and, if considered necessary to avoid isomerization of the reagent 7-AVNA double bond, a catalytic quantity, not greater than 10 mol% on the 7-AVNA, can be added of an acid chosen from alkylsulphonic acids, arylsulphonic acids, 98 wt% sulphuric acid and chlorotrimethylsilane.

Then, allowing the temperature to rise spontaneously to a maximum of +40°C, a silylating agent chosen from N,O-bistrimethylsilylacetamide, N,N'-bistrimethylsilylurea, hexamethyldisilazane, N-trimethylsilylacetamide, N-(tertbutyldimethylsilyl)acetamide, 1-(trimethylsilyl)imidazole, tert-butyldimethylsilyltrifluoroacetamide is added in a quiantity of at least one molar equivalent with respect to the 7-AVNA. The mixture is then left to react for a time between 10 minutes and 240 minutes, until complete transformation of the 7-AVNA into the compound of formula (IV). While maintaining the temperature between +15°C and +40°C, the intermediate (II) is added to the obtained solution of (IV) and allowed to react within this temperature range for a time between 4 and 30 hours.

On termination of the reaction a quantity of decolorizing carbon variable between 5 wt% and 15 wt% with respect to the 7-AVNA used can be added, leaving the mixture under agitation for a further 15-30 minutes, then filtering and slowly pouring the hence decolorized reaction solution, over 30-60 minutes, into an agitated water volume, variable between 5 and 10 times the total volume of the reaction solvent used, at a temperature between +5°C and +40°C.

The obtained suspension with the precipitate is agitated for a further 30-90 minutes, after which the product (V) is isolated by filtration and dried under reduced pressure to constant weight.

The product (V) can be transformed to cefdinir by various known methods, commonly used for the hydrolysis or deprotection of functional groups binding a tert-butyl or a trityl, for example by using Lewis acids or Bronsted acids, in the presence or absence of phenol or anisole, such as boron trifluoride, aluminium trichloride, perchloric acid, trifluoroacetic acid, hydrochloric acid, hydrobromic acid and hydroiodic acid; in particular, adaptation of the method described in Bioorganic and Medicinal Chemistry 11(4), 591-600, 2003, which suggests the initial isolation of cefdinir as the salt of trifluoroacetic acid, has proved excellent in terms of the molar yield of the process and the quantity of the cefdinir obtained.

Cefdinir, as the salt of trifluoracetic acid, isolated from the hydrolysis reaction environment by simple filtration, is added, either solid as such or predissolved in a suitable aprotic organic solvent, into an adequate water volume, i.e. 5-40 volumes with respect to the weight of the corresponding product (V) which generated it and at a temperature between +2°C and +40°C, into which sodium hydrogencarbonate or another carbonic acid salt of an alkaline or alkaline-earth metal has been previously added to the extent of 2-10 molar equivalents with respect to the product (V) used to generate the cefdinir.

In this manner an aqueous solution of cefdinir is obtained as the carboxylate of an alkaline or alkaline-earth metal, of pH between 4.0 and 8.5, which can be washed with water-immiscible aprotic solvents such as ethylacetate and/or dichloromethane and decolorized with a carbon quantity of 5 wt% - 15 wt% with respect to the weight of the substrate (V). If necessary, a reducing species can be added to this solution in a catalytic quantity of 0.001-0.1 molar equivalents with respect to the substrate (V) used, which can be of inorganic nature, such as a sulphurated reducing species of an alkaline or alkaline-earth metal, chosen from a sulphite, a metabisulphite, a hydrosulphite, a thiosulphite, or an organic reducing species such as ascorbic acid or a salt thereof with an alkaline or alkaline-earth metal. The solution obtained by treating in this manner can also be subjected to evaporation under reduced pressure at a temperature between +15°C and +40°C, to eliminate any organic solvent residues present, which could negatively interfere with the isolation of the cefdinir in one of its possible defined crystalline forms. The product is finally precipitated from this solution, at a temperature between 0°C and +40°C, by acidification with an inorganic acid, such as hydrochloric acid, sulphuric acid or phosphoric acid, or an organic acid such as formic acid to a pH between 1.5 and 3.5. The cefdinir is finally recovered by filtration and dried under reduced pressure. From the cefdinir-rich aqueous solution, various crystalline forms can be obtained as the salt of an alkaline or alkaline-earth metal, including the crystal A claimed in US4935507 and the crystal B claimed in patent application US2005/0209451A1, by the methods of the experimental examples described in those documents.

It has therefore been verified that cefdinir can be obtained from the intermediate of formula (V) by using a trifluoroacetic acid quantity, variable between 4 and 65 molar equivalents with respect to the substrate (V), dissolved in dichloromethane, conducting the reaction at a temperature between -15°C and +25°C, in the presence of triethylsilane in a quantity variable between 1 and 20 molar equivalents with respect to the substrate (V) within a reaction time between 10 and 240 minutes.

Proposed experimental examples, non-limiting of the invention, are described hereinafter.

### EXAMPLE 1

### Preparation of (Z)-(2-aminothiazol-4-yl)-2-trityloxyiminoacetic acid N,N-dimethylacetamide solvate.

(Compound of formula (III) with R1 equal to phenyl, y equal to 1 and SOLV' equal to N,N-dimethylacetamide).

139g of (Z)-(2-aminothiazol-4-yl)-2-trityloxyiminoacetic acid, obtained as described in US5637721 but not dried in the presence of P₂O₅ , with a total water content by Karl Fischer titration (from here onwards this titration will be known for short as "K.F.") of about 10%, are suspended in 264 ml of N,N-dimethylacetamide under agitation, then heating to T = +50°C without visible solubility; agitation is applied at this temperature for 30-45 minutes.

The mixture is cooled to T = +15°C, and 400 ml of diisopropylether are added over about 30 minutes. The temperature is further lowered to T = 0°C and agitation applied at this temperature for 1 hour. The mixture is filtered and filtrate portions are washed with 300 ml of diisopropylether. Drying is applied at T = +55°C under reduced pressure to constant weight. 144 g of product are obtained as a white solid.

¹HNMR-DMSO-d₆ : by integrating signals at 2.92 ppm, 2.77 ppm and 1.94 ppp the product is a dried N,N-dimethylacetamide monosolvate with K.F. less than or equal to 1.0%.

### EXAMPLE 2

### Preparation of diethylphosphoryl (Z)-2-(2-aminothiazol-4-yl)-2-trityloxyiminoacetic acid dichloromethane hemisolvate.

### (Compound of formula (II) with R1 equal to phenyl, X equal to sulphur and m equal to 0.5).

100 g of the product obtained in Example 1 are suspended at T = =20°C in 1000 ml of dichloromethane and 0.217 g of 1,4-diazabicyclo[2.2.2]octane are added. At T = +15°C/+20°C 46.6 g of tributylamine are rapidly dripped in and agitation is applied for 15 minutes at T = +20°C until dissolution. The mixture is cooled to T = 0°C°C/+5°C. It is filtered, washing the filtrate portions with 200 ml of dichloromethane precooled to T = 0°C. The product is dried under reduced pressure at +25°C to constant weight. 118.0 g of product are obtained as a white solid.

### EXAMPLE 3

### Preparation of 7β-[(Z)-2-(2-aminothiazol-4-yl)-2-trityloxyiminoacetamide]-3-vinyl-3-cefem-4-carboxylic acid 1/3 diethylthiolphosphate N,N-dimethylacetamide monosolvate.

### (Compound of formula (V) with R1 equal to phenyl, R' equal to ethyl, X equal to sulphur, k equal to 1/3, n equal to 1 and SOLV equal to N,N-dimethylacetamide).

1132.8 g of 7-amino-3-vinyl-3-cefem-4-carboxylic acid in 705 ml of N,N-dimethylacetamide at T = +15°C. 3.5 g of 98 wt% sulphuric acid are slowly dripped in. Agitation is applied at this temperature for at least 1-2 minutes. 119.2 g of N,O-(bistrimethylsilyl)acetamide are added over 10 minutes, being careful not to exceed T = +30°C. The mixture is agitated for 3 hours or until a clear solution is attained.

391.0 g of the product obtained in Example 2 are added and left to react at T = +24°C/+26°C for 22 hours. 16.6 g of carbon are then added for decoloration and agitation applied at T = +24°C/+26°C for a further 15 minutes. The carbon is filtered off, and the filter washed with a total of 360 ml of N,N-dimethylacetamide in portions. The thus decolorized solution is added over about 40 minutes to 6250 ml of demineralized water agitating strongly at T = +15°C/+20°C. The agitation is slowed down and the T maintained at +20°C for about one hour. The mixture is filtered and the filtrate washed with 3150 ml of demineralized water in portions. The product is dried under reduced pressure at T = +30°C/+35°C, to obtain 453.0 g of product.

### EXAMPLE 4

### Preparation of cefdinir both in crystalline form A and in crystalline form B.

30 g of the product synthesized as in Example 3 are added to a solution cooled to T = -15°C, consisting of 180 ml of dichloromethane, 80.3 g of trifluoroacetic acid and 112.0 g of triethylsilane, taking care not to exceed a temperature of -5°C. The mixture is then left to react for about 60 minutes at T = 0°C/-5°C, resulting in an abundant precipitate which is filtered off and washed with 4 x 25 ml portions of dichloromethane precooled to 0°C. The thus filtered product, as cefdinir trifluoracetic acid salt, is dissolved in a solution consisting of 100 ml of acetone and 100 ml of ethyl acetate at ambient temperature. 3 g of carbon are added to the solution obtained, agitating at ambient temperature for 10 minutes. The carbon is filtered off and the filter washed with 50 ml of acetone and then with 50 ml of ethyl acetate.

The decolorized solution is slowly added to a suspension of 23 g of sodium hydrogen carbonate in 250 ml of demineralised water at T = +15°C/+25°C, taking care never to lower the solution pH below 7.2 during the operation.

The two-phase solution is separated and the underlying aqueous phase, in which the product is dissolved, is washed with 150 ml of dichloromethane. The residual solvents present in solution are eliminated by evaporating under reduced pressure while maintaining the solution temperature around +20°C/+25°C. The temperature is raised to +30°C/+35°C, and at this temperature, which is maintained until termination of the process, 15% w/w aqueous hydrochloric acid is dripped in under fast solution agitation at 300-400 r.p.m. until incipient product precipitation, at pH = 3.5/3.8. The mixture is left under fast agitation for 15 minutes, taking care to maintain the pH value between 3.5-3.8 by adding a few drops of 15% w/w hydrochloric acid. 15% w/w is then dripped in under sustained agitation until pH 1.8/2.0. The mixture is finally agitated for 15-30 minutes, the product is filtered off and washed with 50 ml of demineralised water. The product is dried under reduced pressure at T = +30°C/+35°C. 10.3 g of cefdinir are obtained as crystal A (K.F. = 0.73%/titre for untreated sample 95.5%) with X-ray diffractogram superposable on that described in US4935507, (K.F. = 0.72/ titre for untreated sample 94.9%), with X-ray diffractogram superposable on that described in US4935507.

Alternatively, 4.2 ml of triethylsilane are added to a solution cooled to T = -5°C formed from 45 ml of dichloromethane and 13.1 ml of trifluoroacetic acid. 7.5 g of the product synthesized in Example 3 are added in portions to the solution obtained, cooled to T = -15°C, taking care not to exceed the temperature of -5°C.

The mixture is then left to react for about 60 minutes at T = +0°C/- 5°C resulting in an abundant precipitate which is filtered off with 4 x 6 ml portions of dichloromethane precooled to 0°C. The thus filtered and squeezed product is slowly added to a suspension of 5.75 g sodium hydrogen carbonate in 63 ml of demineralised water at T = +15°C/+25°C, taking care never to lower the solution pH below 7.20 during the operation.

The solution, in which the product is dissolved, is decolorized with 0.75 g of carbon, filtering off the carbon and washing the filter twice with 10 ml of demineralized water each time. The decolorized solution is then washed in succession with 37 ml of ethyl acetate and 37 ml of dichloromethane. The residual solvents present in the aqueous solution are eliminated by evaporating under reduced pressure while maintaining the solution temperature around +20°C/+25°C. The temperature is lowered to +0°C/+5°C, and at this temperature, which is maintained until termination of the process, 15% w/w aqueous hydrochloric acid is dripped in very quickly while agitating the solution, until the pH lies between 2.0 and 2.2 and an abundant precipitate forms. The product is finally agitated for 15-30 minutes, washing with 12.5 ml of demineralised water precooled to T = +0°C/+5°C. The product is dried under reduced pressure at T = +30°C/+32°C. 2.41 g of cefdinir are obtained as crystal B (K.F. = 5.82%/titre for untreated sample 89.7%) with X-ray diffractogram superposable on that described in US2005/0209451A1.

## Claims

1. A process for obtaining cefdinir of formula (I) 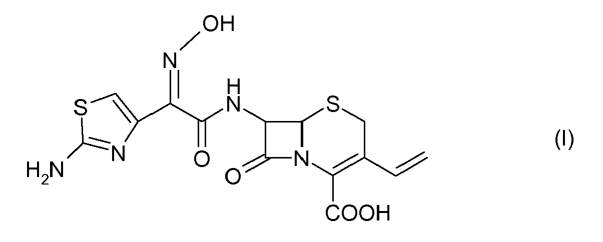 according to which a compound of formula (II) 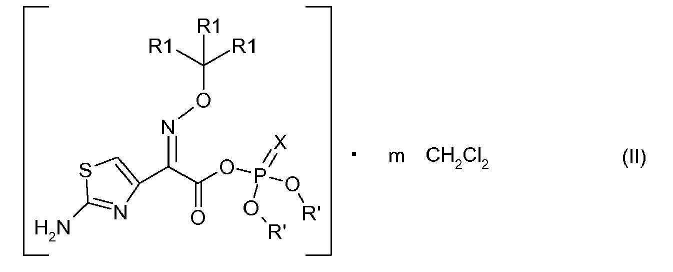 in which the two substituents R1 and R' are independently chosen from the group consisting of phenyl, C1-C4 alkyls, X is chosen from the group consisting of oxygen and sulphur, 0 ≤ m ≤ 1, a compound which in its turn is obtained from the corresponding carboxylic acid of formula (III), 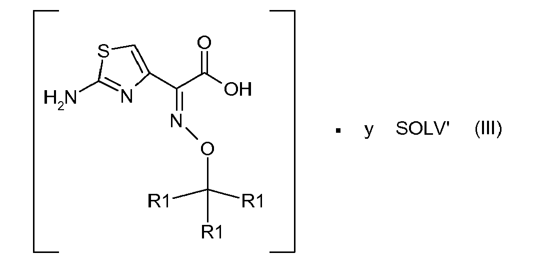 in which SOLV' is a solvent chosen from the group consisting of N,N-dimethylacetamide, N,N-dimethylformamide, N-methylformamide, N-methylacetamide, N-methylpyrrolidone and formamide, R1 has the same meaning expressed in formula (II) and 0 ≤ y ≤ 2, is condensed with a silylester of formula (IV) 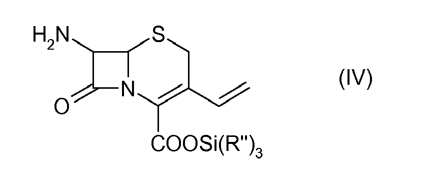 where R" is chosen from the group consisting of equal or different C1-C4 alkyls, to obtain an intermediate, isolated from an aqueous solution which is finally reacted with an acid chosen from the group consisting of borotrifluoride, trifluoroacetic acid, aluminium trichloride, perchloric acid, hydrochloric acid, hydrobromic acid and hydroiodic acid, to generate cefdinir in the corresponding salified form from which cefdinir is obtained.

2. A process as claimed in claim 1, wherein said salified form of cefdinir, obtained by filtration from the reaction environment, is then added to a volume of water in which a carbonic acid salt has been previously dissolved, to hence obtain an aqueous solution of cefdinir, of pH between 4.0 and 8.5, which is washed with water-immiscible aprotic organic solvents and decolorized with carbon, to which there has been added a reducing species chosen from the group comprising sulphurated reducing species, themselves chosen from a sulphite, a metabisulphite, a hydrosulphite and a thiosulphite of an alkaline or alkaline-earth metal, organic reducing species and ascorbic acid, or a salt thereof of an alkaline or alkaline-earth metal, to finally precipitate the product at a temperature between 0°C and +44°C, by acidification to a pH between 1.5 and 3.5, then filtering the cefdinir precipitated in this manner and drying it under reduced pressure.

3. A process as claimed in claim 1, wherein the cefdinir is obtained by alkalination of its corresponding salified form in solution, followed by subsequent acidification.

4. A process as claimed in claim 1, wherein the cefdinir is obtained in crystalline form.
